(19) Europäisches Patentamt
European Patent Office
Office européen des brevets

(11) Publication number: **0 137 644**
**B1**

(12) **EUROPEAN PATENT SPECIFICATION**

(45) Date of publication of patent specification: **08.08.90**

(51) Int. Cl.$^5$: **A 61 F 13/15,** A 61 L 15/16

(21) Application number: **84305522.9**

(22) Date of filing: **14.08.84**

(54) **Corrugated web structure.**

(30) Priority: **15.08.83 US 523473**
**15.08.83 US 523474**
**15.08.83 US 523501**

(43) Date of publication of application:
**17.04.85 Bulletin 85/16**

(45) Publication of the grant of the patent:
**08.08.90 Bulletin 90/32**

(84) Designated Contracting States:
**AT BE CH DE FR IT LI LU NL SE**

(56) References cited:
**DE-A-2 062 790**
**FR-A-2 504 799**
**GB-A-2 087 240**
**US-A-3 525 337**
**US-A-4 111 733**
**US-A-4 354 901**

(73) Proprietor: **PERSONAL PRODUCTS COMPANY**
**Van Liew Avenue**
**Milltown New Jersey 08850 (US)**

(72) Inventor: **Holtman, Dennis C.**
**Box 125 RD 5 Old Clinton Road**
**Flemington New Jersey 08822 (US)**

(74) Representative: **Jones, Alan John et al**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 137 644 B1

**Description**

The present invention relates to new and improved corrugated web structures and in addition to new and improved absorbent corrugated web structures incorporating therein superabsorbent.

Disposable absorbent products have been known for some time, including such products as disposable diapers, sanitary napkins, wound dressings, bandages and incontinence pads. These products incorporate an absorbent batt which is used to absorb and hold or contain body fluids. Initially in many of these products, especially diapers and sanitary napkins, the absorbent batt comprised what is termed "wadding" or plies of tissue. The wadding was disposed between a liquid-impermeable backing and a liquid-permeable facing and the plies of tissue were used to absorb and, hopefully, contain the liquid within the product. A diaper which utilizes such an absorbent batt is disclosed in US—Re—26,151.

The wadding type of product was replaced, for the most part, by an improved absorbent batt which comprises what is termed "fluffed woodpulp fibers". This absorbent batt comprises a layer of individualized woodpulp fibers with the layer having substantial thickness. A diaper which incorporates such a fluffed woodpulp absorbent batt is described in US—A—2,788,003. This diaper had improved absorbent capacity and somewhat better containment than a diaper using a wadding layer. Also, the fluffed woodpulp layer is quite soft, flexible, and conformable, and, hence, produces an improved diaper over diapers using wadding as the absorbent layer.

Though the fluffed woodpulp absorbent batts have improved capacity, the efficiency with which the capacity is used in a diaper or sanitary napkin is poor. The reason for this is that the fluid to be absorbed is generally deposited in a localized area within the absorbent batt, and the ability of the fluid to move along the plane of the batt is poor. The fluid tends to follow a radial wicking path and consequently moves to the closest edge of the batt where it generally is no longer contained and the product leaks.

US—A—3,017,304 discloses an absorbent product which incorporates in the product a densified paper-like layer. This paper-like layer acts as a wick, i.e. liquid which is placed on the layer tends to move rapidly along the plane of the layer. When incorporated in combination with fluffed woodpulp fiber, the resultant product uses the absorbent capacity of the fluffed woodpulp much more efficiently. Diapers which incorporate this paper-like layer combined with fluffed woodpulp are disclosed and described in US—A—3,612,055 and US—A—3,938,522. This concept of combining wicking ability, or a capillary skin or layer, with fluffed woodpulp fibers has gained wide acceptance in many absorbent products including disposable diapers and sanitary napkins. Even though these products make much greater use of the capacity of the absorbent batt, they still do not totally contain the absorbed liquid. It is probable that these products will leak before the full capacity of the batt is used for the absorption or, at the very least, before the entire liquid void by the user is absorbed. This is especially true when pressure is placed on the batt while wet. For example, a baby sitting down on a previously wetted diaper will very often cause the batt to leak.

An incontinent adult faces not only the problems of the infant but many other problems. First, the void of an adult generally is much higher in volume than that of an infant. Second, a bulge under clothing is accepted by society for an infant, but the ambulatory adult with an incontinence problem longs for a product which is not visible through ordinary clothing. Third, the proportions and shape of the legs and torso of the adult differs considerably from those of an infant. Therefore, a mere enlargement of an infant diaper, such as that shown in US—A—4,253,461, is not a satisfactory product.

In both the infant diaper and adult incontinence product marketplace, a product is needed which has a large storage capacity. For instance, shaped containers have been suggested. However, these containers have been substantially rigid, do not stay in place, and are quite uncomfortable. A product with a substantially large storage capacity, with an ability to move liquid away from the void zone, which is disposable, which is comfortable, and which does not show through wearing apparel is needed in the marketplace.

A number of years ago, "superabsorbent materials", i.e. materials which will absorb many times their weight of liquid, were developed. Since the development of such materials, attempts to incorporate them in absorbent products, such as diapers, to enhance the absorption performance of these products have been made. Theoretically, a minimum amount of superabsorbent incorporated in a product would make that product perform as well or better than the prior art products. Perhaps one of the first products to incorporate such a superabsorbent material in a disposable diaper is disclosed in US—A—3,670,731. This patent discloses an absorbent dressing comprising an absorbent layer sandwiched between a permeable facing and an impermeable backing sheet. The absorbent layer contains water-insoluble cross-linked hydrocolloid polymer as the superabsorbent material.

Even though superabsorbent materials have been available for some time, they have not gained wide acceptance in absorbent products such as disposable diapers and sanitary napkins. A primary reason for this lack of acceptance of superabsorbents is failure to develop a product capable of economically utilizing the highly increased absorptive capacity of the superabsorbent material. In order to utilize economically a superabsorbent, the liquid being absorbed must be readily accepted and placed in contact with the superabsorbent material. Furthermore, as the superabsorbent material absorbs liquid, it must be allowed to swell. If the superabsorbent is prevented from swelling, it will cease absorbing liquid. Hence, if the superabsorbent material is to function in absorbent products, such as diapers and sanitary napkins,

wherein the liquid to be absorbed is placed in a small void area, the structure of the absorbent layer containing superabsorbent materials must have certain characteristics. Over the years, a number of techniques have been disclosed in an attempt to provide structures which make efficient use of the superabsorbent material. Such products are disclosed in US—A—4,103,062; US—A—4,102,340; and US—A—4,235,237. In addition, methods for incorporating superabsorbents into suitable layers or suitable configurations which can be placed in an absorbent product, are disclosed in US—A—4,186,165; US—A—4,340,057; and US—A—4,364,992. To date, none of these products have met with any substantial commercial success.

A corrugated absorbent product is disclosed in US—A—3 525 337. The product consists of an accordion pleated pad formed from a thin layer of absorbent fibers faced on each side with sheets of absorbent product comprising a first layer and a second layer discrete from but united to said first layer, wherein:

the first layer comprises a non-woven web comprising hydrophobic, resilient, synthetic fibers;

the second layer has a higher capillary pressure than that of the first layer to provide preferential attraction and wicking of liquid in the second layer;

the layers are transversely folded to provide a corrugated structure; and

the corrugated structure is stabilised to retain its transverse folds when wet,

characterised in that the web is stabilised by face-to-face bonding between corrugations.

According to a second aspect of the present invention, there is provided an absorbent product comprising:

a corrugated non-woven web comprising hydrophobic, resilient, synthetic fibres, stabilised to retain its transverse folds when wet, and

at least 10% by weight of the web of an absorbent associated with the web,

characterised in that:

the web is stabilised by face-to-face bonding between corrugations; and

the absorbent is a superabsorbent.

The present invention provides a new and improved absorbent product which possesses a large storage capacity, which is soft and comfortable, which can be designed so as not to be apparent through normal clothing, and which, if desired, utilizes a substantial portion of the absorptive capacity of superabsorbent materials. In addition, the new absorbent product will contain absorbed liquid even when pressure is placed upon the product during use.

In its first aspect, the present invention provides a disposable absorbent product comprising a first fibrous layer, in the form of a nonwoven web. A second fibrous layer discrete from the first layer but united to the first layer is provided which second layer has a higher capillary pressure than that of the first layer. This provides preferential wicking of liquid in the second layer. These layers, in their united form, are transversely folded to provide a corrugated structure which is stabilized by face-to-face bonding in such a way as to retain its transverse folds even when wet.

In its second aspect, the present invention also provides an absorbent product comprising a corrugated fibrous web which contains at least 10% by weight of superabsorbent preferably about 50—90%. The corrugated web is stabilized by face-to-face bonding to retain its transverse folds even when wet. The absorbent product may be stabilized by adding a minor portion of fibers having a lower melting point than the remaining fibers in the fibrous web and subjecting the corrugated web to temperatures sufficient to melt the fibers, thereby providing heat bonding between the folds or within the corrugations.

The products of the present invention may further comprise a liquid-impermeable barrier covering at least one side of the web, and a liquid-permeable facing covering at least the other side of the web.

The first fibrous layer comprises substantially hydrophobic, resilient, preferably synthetic fibers, in the form of a nonwoven web. The second layer, if present, is comprised of fibers (or in the case of peat moss, particles) which when placed in the form of a layer provides a higher capillary pressure than the capillary pressure of the first fibrous layer. As a result, the second layer drains liquid from the first layer and wicks the liquid away from the void zone.

The superabsorbent may be of a wide particle size range and is distributed in any one of a number of ways, e.g. as a layer, or film or as individual particles or globules or as part of the web being corrugated, and is associated either with the first fibrous layer, or, if present, with the second layer.

In a preferred embodiment, the first fibrous layer is prepared in the form of a nonwoven web and the second layer is deposited by known procedures onto the first layer. The second layer is comprised of a wicking substance which provides a higher capillary pressure than that of the first layer. The wicking substance includes hydrophilic fibers such as cellulosic fibers, rayon fibers and other wicking substances such as peat moss or mixtures thereof or acrylic fibers or the like. The wicking substance which forms the second layer generally is comprised of fibers or particles in closely spaced relationship to promote the movement of liquid along the second layer.

When the absorbent product is utilized after the transverse folding or corrugation takes place, the liquid preferentially moves along the second layer whether the second layer is vertical or horizontal. The corrugations are placed in an absorbent structure, such as a diaper or incontinence product, so as to lie parallel to the longitudinal axis of the product.

Body fluids such as urine, menstrual fluid or other fluids are deposited in a localized area on the first

fibrous layer in any given area of the absorbent product. The second layer immediately commences its draining and transporting activity to remove the liquid from the localized area. As the liquid front moves horizontally, it also moves vertically, thus gradually being transferred from one transverse fold to another.

In another embodiment, the absorbent product is prepared by placing superabsorbent on the fibers of the web prior to corrugation, corrugating the web, and then stabilizing the web to retain its transverse folds even when wet.

In the accompanying drawings:

Figures 1 and 1A are side elevational views of portions of suitable webs for use in the present invention;

Figures 2 and 2A are side elevational views of the starting material of Figures 1 and 1A respectively after transverse folding has begun taking place;

Figure 3 is a side elevational view of the material of Figure 2 after corrugation and stabilizing has taken place;

Figure 4 is a perspective view illustrating one embodiment of the present invention;

Figure 5 is a side elevational view of a portion of another starting material for use in the present invention;

Figure 6 is a side elevational view of the starting material of Figure 5 as transverse folding is taking place;

Figure 7 is a perspective view of a specific embodiment of the present invention;

Figure 8 is a perspective view of another embodiment of the present invention;

Figure 9 is an enlarged cross-sectional view through lines 10—10 of Figure 8 depicting three different internal webs;

Figure 10 is a perspective view of a further embodiment of the present invention; and

Figure 11 is a cross-sectional view through lines 12—12 of Figure 10, depicting three different internal webs.

Referring now to the drawings, Figure 1 represents a side elevational view of a segment of starting material 10 depicting a fibrous layer 12 having distributed therein superabsorbent 14.

Figure 2 depicts the starting material 20 in partially corrugated form wherein a fibrous web 22 contains superabsorbent 24.

Figure 3 is a side elevational view of the starting material of Figure 1 after corrugation to provide a corrugated segment of the starting material 30. The starting material 30 contains a fibrous web 32 having distributed therein superabsorbent 34 and is bonded at points 36 wherein fusible fibers have been permitted to melt to make bonding contact points. Thus, the starting material 30 is corrugated and stabilized so as to maintain its transverse folds even when wet.

Figure 1A represents a side elevational view of a segment of starting material 10A depicting a first fibrous layer 12A and a united second layer 14A prior to a transverse folding or corrugation treatment of the starting material 10A.

Figure 2A provides a side elevational view of the starting operation of corrugating the starting material 20A. The starting material 20A consists of a nonwoven fibrous web 22a, and a second layer 24A of fibers wherein the density is greater than that of the fibrous layer 22A. This starting material 20A can be face-to-face bonded after corrugation to provide a product according to the present invention.

Figure 4 is a perspective view of a corrugated web product 40 of the present invention. The corrugated web product 40 contains a superabsorbent distributed throughout the web and is stabilized as shown in Figure 3.

Figure 5 is a side elevational view of a segment of starting material 50 which is a fibrous web 52 but not containing any superabsorbent.

Figure 6 depicts a segment of starting material 60 such as that in Figure 5 in a partially corrugated form wherein superabsorbent 64 has been placed in the pockets of the partially corrugated web 62. This starting material 60 containing the superabsorbent 64 can be face-to-face bonded after corrugation to provide a product according to the present invention.

Figure 7 depicts a disposable diaper 80. A moisture-pervious facing 82, such as a nonwoven fabric, provides the diaper surface. A moisture-impervious substance 84, such as polyethylene, forms the moisture-proof backing of the diaper. The diaper structure 80 contains an absorbent product 83 sandwiched between the facing 82 and the backing 84. The absorbent product 83 is that described and shown in Figure 4. The diaper side edges are gathered in the crotch region by elastic members 87. To secure the diaper about the waist of the wearer, tape tabs 89 are provided. The diaper product 80 generally has the absorbent product 83 placed in such a manner that the corrugations run parallel to the longitudinal axis of the product.

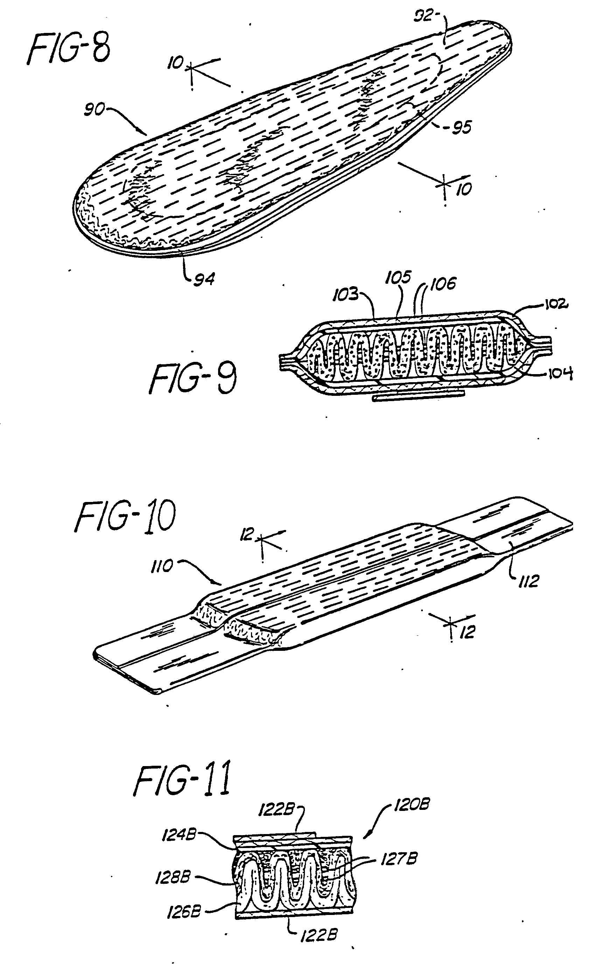

Referring now to Figure 8, a urinary pad 90 is depicted. The urinary pad 90 has a moisture-permeable facing 92 covering the entire upper surface. Immediately beneath the facing 92 is a liquid barrier 94 which encompasses the entire product except for the opening 95 on the upper surface which lies immediately below the facing 92. The opening 95 permits ingress of fluid.

Figure 9 is an enlarged cross-sectional view of Figure 8 taken along lines 10—10. The facing 102 is the layer which is placed against the skin of the wearer. The liquid barrier 104 encompasses the absorbent

product 103 except for the opening 105 wherein fluid is permitted to enter. The absorbent product 103 is similar to that depicted in Figure 4.

Figure 10 illustrates a sanitary napkin 110 having a fabric overwrap 112 which is liquid permeable.

Figure 11 depicts an enlarged cross-sectional view of Figure 10. The cross-sectional portion of product 120B is provided with a liquid-permeable overwrap 122B appearing with its overlapped portion on the upper surface of the drawing. Immediately below is a moisture-impermeable barrier 124B which encompasses the sides and the bottom of the product. The corrugated web consists of a fibrous web layer 126B and another layer 128B which layer has a higher capillary pressure than the fibrous web layer 126B. Superabsorbent 125B is placed in the pockets created by the base of the corrugations of the web and the liquid-impermeable backing 124B. The superabsorbent is substantially trapped in this pocket. The overwrap 122B provides the facing on the lower surface in the drawing. The facing 122B is placed against the skin of the wearer.

These and other products such as incontinence pads, wound dressings and wipes, may be made from the absorbent product depicted in Figure 4 or segments of which are depicted in Figure 3.

The first fibrous web used to provide the corrugated fibrous web is generally of substantially high loft and upon dry compression followed by release has a tendency to return substantially to its original thickness. For instance, fibrous webs formed from synthetic fibers such as polyethylene, polypropylene, polyester, nylon (polyamide fibers), bi-component fibers or mixtures thereof are particularly suitable. Generally, the fibers are carded to form a web which is then stabilized if needed. Stabilization may be achieved by heat-through bonding, adhesive bonding, point embossing with heat or adhesive or both. The stabilizing process is selected according to the fibers used and the process used to form the web. Other suitable procedures for forming a web including air-laying, wet-laying, spun bonding, laying of melt-blown fibers and other known techniques. The fibrous web, before corrugation, preferably has a dry bulk of at least about 10 cc per gram, and a weight less than 4 oz. per square yard (135.6 g/m$^2$).

In one embodiment, wherein superabsorbent is used, a blend of staple polyester fibers with a minor portion of fusible fibers, such as lower melt polyester fibers, are carded to form a web. The web is subsequently lightly bonded by passing hot air through the fibers making the fusible fibers tacky so as to stick to each other and the staple fibers to provide the desired degree of integrity to the web structure.

The superabsorbent, present either on the fibers of the web or placed in the folds of the corrugated web, or otherwise associated with the web, is generally a water-insoluble, water-swellable polymeric substance capable of absorbing water in an amount which is at least 10 times the weight of the substance in its dry form. The superabsorbent is in the form of fibers, spheres, particles, bits of film, globules, webs or film, or may be applied in the form of a liquid monomer solution which is subsequently polymerized. The superabsorbent prepared by polymerization of a monomer solution placed on fibers in a web is most frequently in the form of globules and bits of film-like particles in the web structure.

One type of superabsorbent material provides particles or fibers which may be described chemically as having a backbone of natural or synthetic polymers with hydrophilic groups or polymers containing hydrophilic groups being chemically bonded to the backbone or in intimate mixture therewith. Included in this class of materials are such modified natural and regenerated polymers as polysaccharides including, for example, cellulose and starch and regenerated cellulose which are modified by being carboxyalkylated, phosphonoalkylated, sulfoalkylated, or phosphorylated to render them highly hydrophilic. Such modified polymers may also be cross-linked to improve their water-insolubility.

These same polysaccharides may also serve, for example, as the backbone on to which other polymer moieties may be bonded by graft copolymerization techniques. Such grafted polysaccharides and their method of manufacture are described in US—A—4,105,033 and may be described as polysaccharide chains having grafted thereon a hydrophilic chain of the general formula:

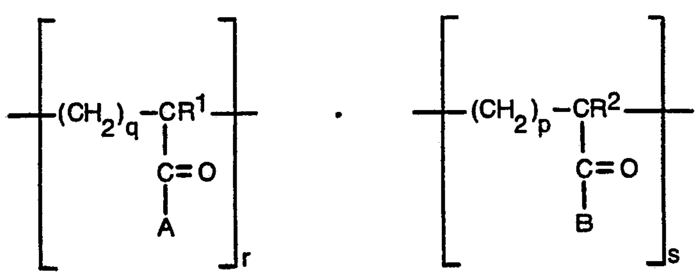

wherein A and B are selected from the group consisting of —OR$^3$, —O(alkali metal), —OHNH$_3$, —NH$_2$, wherein R$^1$, R$^2$, and R$^3$ are selected from the group consisting of hydrogen and alkylene having 1 to 4 or more carbon atoms wherein r is an integer having a value of 0 to about 5000 or more, s is an integer having a value of 0 to about 5000 or more, r plus s is at least 500, p is an integer having a value of 0 or 1, and q is an integer having a value of 1 to 4. The preferred hydrophilic chains are hydrolyzed polyacrylonitrile chains and copolymers of polyacrylamide and polysodium acrylate.

In addition to the modified natural and regenerated polymers, the hydrocolloid component may comprise wholly synthetic hydrophilic particles. Examples of those now known in the art are polyacrylonitrile fibers which may be modified by grafting moieties thereon such as polyvinylalcohol chains, polyvinyl alcohol itself, hydrophilic polyurethane, poly(alkyl phosphonates), partially hydrolyzed polyacrylamides (e.g. poly(N-N-dimethylacrylamide), sulfonated polystyrene, or a class of poly(alkyleneoxide). These highly hydrophilic synthetic polymers may be modified by other chemical treatments such as cross-linking or hydrolysis. Further examples known in the art are the non-ionic polymers such as polyoxyethylene, polyoxypropylene, and mixtures thereof which have been suitably cross-linked, either chemically or by irradiation. Still another more recent type is a derivative of isobutylenemalic and acrylate monomers, such as sodium, potassium, ammonium, (or a combination of cations) acrylate, may be placed on the absorbing layer by spraying or otherwise placing a solution thereon, followed by polymerization and cross-linking, for example by irradiation.

In addition, naturally occurring materials such as gums may be used. Examples of such suitable gums include guar gums, acacia gums and locust bean gums.

The superabsorbent is combined with the web in such a manner as to remain substantially in the same position or region even though the web may be moved about during manufacturing, packaging, or use. The superabsorbent is combined with the fibrous web either before or after corrugation by any means suitable preferably to place the superabsorbent so as to try to minimize interference by one superabsorbent particle or fiber with another upon the swelling of the first. If the superabsorbent material is a powder, it may be sprinkled on to the fibrous web either in dry form or the web may be moistened or the powder may be placed into a transverse fold of the corrugated web. If the superabsorbent is in granular form, it may be desirable to slightly moisten the superabsorbent before placing it in contact with the web. The superabsorbent generally will be in the form of particles or fibers or globules which may range in size from about 0.0005 mm in diameter to globules that are continuous along fibers for a distance of several inches (centimeters).

Another method of placing superabsorbent in a fibrous web is by spraying a monomer solution on the web or perhaps even saturating the web with a monomer solution, followed by polymerization of the monomer. One typical way to polymerize the monomer is by use of irradiation. It is desirable to place a superabsorbent somewhat evenly throughout the fibrous web. However, even if the superabsorbent is powder-like and in the form of a layer, it tends to function better in the present corrugated web than in previously known products.

Any superabsorbent which absorbs large amounts of liquids is suitable for use in the absorbent product of the present invention.

Corrugating or transverse folding of the web is carried out by known procedures such as that exemplified in US—A—4,111,733. The web corrugations range from three to six or even eight per inch (2.54 cm) of corrugated web. The corrugated web is generally from 1/4 to 3 inches (0.63 to 7.62 cm) preferably from 1/2 to one inch (1.27 to 2.54 cm) thick.

After or during corrugating of the fibrous web, the corrugated structure is stabilized to prevent the corrugations from pulling apart and flattening out either in the dry form or when becoming wet. One method of stabilizing the web is accomplished by using an adhesive binder which may be a latex resin or other known adhesive.

Another method of stabilizing the web is by adding a small portion of fusible fibers to the web fibers before the web is made. These fusible fibers have a lower melting point than the remaining fibers and when the corrugated web is subjected to temperatures sufficient to melt the fusible fibers, light bonding is provided between the corrugations.

In order for the fibrous web to provide the most desired medium for receiving and holding liquid, it is preferred that the fibrous web have a dry bulk of at least about 10 cc per gram and a weight of less than about 4 ounces per sq. yd. (135.6 g/m$^2$) prior to corrugation preferably from about 1—2 ounces per sq. yd. (33.9 to 67.8 g/m$^2$). The dry bulk is the area times thickness of the web (prior to corrugation) under a load of 0.01 psi (68.95 Pa) calculated in cubic centimeters. This value is divided by the weight in grams in order to provide the measurement in cubic centimeters per gram. It has been found that using a corrugated web as the provider of void volume to contain body fluids has many advantages. For instance, fibers may be used to form the web that in the non-corrugated web form do not have enough wet resilience to retain void volume when the web becomes wet. Corrugating of the web provides the highly desirable resilience in the product that is required to initially accept and hold a high volume of fluid. Also it has been found that superabsorbent may be randomly distributed in small or large quantities within the web with surprisingly high utilization of the superabsorbent. It is theorized that the wet resilience of the corrugated web permits the void volume to remain available almost in totality when large quantities of fluid are present in the web. This would permit the superabsorbent to swell, as it captures the liquid, without substantial inhibition.

In another embodiment of the present invention, an absorbent product is provided which comprises two layers which are corrugated. The product contains the fibrous web previously discussed, and united with it, but yet discrete from it, is a second layer which has a higher capillary pressure than that of the fibrous web to provide preferential attraction and wicking of liquid in the corrugated web product. In other words, in this embodiment the corrugated web consists of two layers, one of which is the fibrous web discussed before, and the other of which is a second layer united with the first layer but discrete from it. The

second layer is comprised of fibers (or in the case of peat moss, particles) which, when placed in the form of a layer, provides a higher capillary pressure than the capillary pressure of the first fibrous layer. As a result, the second layer drains liquid from the first layer and wicks the liquid away from the void zone. The superabsorbent is placed as before so as to be in contact with the second layer; that is, either between the layers before the layers are corrugated or in the pockets of the corrugated web next to the second layer. In addition, the superabsorbent in the present embodiment can be placed between the two layers. In a typical example of preparation of such a structure, the first fibrous layer is formed and superabsorbent is distributed on that layer. The superabsorbent may be in the form of bits of film, particles, globules or powder. The second layer is deposited by known procedures on to the first layer on the side on which the superabsorbent has been distributed. The second layer is united, at least in part, to the first layer by known techniques such as by use of adhesive, by use of vacuum to cause some of the fibers of the second layer to partially integrate with the first layer or by light compression. The two at least partially united layers are corrugated to form the absorbent product. In typical use, the first fibrous layer of the corrugated absorbent product is exposed to the initial impact of the liquid to be absorbed. This initial reception region must be able to accept liquid rapidly and at the same time be able to bear the liquid load even with body weight pressure applied until the second layer with its higher capillary pressure drains a substantial portion of the load and begins wicking the liquid away to another part of the product. The superabsorbent requires time to absorb liquid and swell. The corrugated web holds the liquid and permits the superabsorbent to act.

What appears to be only a small difference in capillary pressure is all that is required for the second layer to attract and drain the first fibrous layer of liquid the latter has received. The force causing a liquid to enter a cylindrical capillary is expressed by the equation

$$P = \frac{(2\gamma \cos \theta)}{r}$$

wherein the force is represented by the capillary pressure and

P is the capillary pressure,

γ is the surface tension of the liquid,

θ is the liquid-fiber contact angle, and

r is the capillary radius.

With a given liquid, the pressure (capillary force) increases with the cosine of the liquid-fiber contact angle (reaching a maximum where the angle is zero) and also increases with narrower capillary radii so that narrower capillaries will draw liquid from wider ones.

The relative wickability between the first fibrous layer and the second layer is affected by both the relative densities of the layers and the relative wettability of the individual fibers in each layer. The individual fibers of the second layer have substantially smaller liquid-fiber contact angles than those of the first fibrous layer overcoming the density difference and providing a significant overall increase in capillary pressure to absorb liquid into the second layer.

The second layer fibers (or particles) and the density of the layer are selected to create a significant difference in capillary pressure from the first fibrous layer.

The second fibrous (or particle) layer is generally comprised of fibers having a lower liquid-contact angle or wherein the layer is provided with narrower capillary radii. Examples of such fibers include hydrophilic fibers such as rayon fibers, cellulosic fibers, or peat moss, or mixtures thereof, or acrylic fibers. Cellulosic fibers include wood pulp fibers and cotton linters.

The wood pulp fibers generally are those that are used to form the fluff or fibrous batt layer in conventional absorbent products such as disposable diapers and sanitary napkins. Other cellulosic fibers that might be used are rayon fibers, flax, hemp, jute, ramie and cotton. The fiber, or peat moss, or mixtures thereof are placed in such a way as to form a layer in which the particles are close to one another so as to promote wicking of liquid in the plane of the layer.

The second layer can be preformed and placed next to the first fibrous layer or the particles (fibers or peat moss or mixtures thereof) can be air-laid, wet-laid, or otherwise combined with the first fibrous layer before the transverse folding or corrugating takes place.

Corrugating or transverse folding of the web, whether it be a single layer or double layer web, is provided by known procedures such as that exemplified in US—A—4,111,733.

After or during corrugating of the web, the corrugated structure is stabilized to prevent the corrugations from pulling apart and flattening out. One method of stabilizing the web is accomplished by using an adhesive binder which may be a latex resin or other known adhesive.

Another method of stabilizing a web is to add a minor portion of fibers having lower melting points than the remaining fibers in the layer and to subject the corrugated web to temperatures sufficient to melt the fusible fibers thereby providing light bonding between the corrugations.

Examples of methods of preparing an absorbent product of the present invention are as follows. These examples are not intended to be limiting in any way and extensions and modifications thereof, without departure from the scope of the invention, will become apparent from these examples.

Example I

A web is formed of polyester fibers by dry-laying the fibers, i.e. by air-laying or carding the fibers to form a web. Specifically, the polyester fibers contain a minor portion of fusible fibers which are also polyester fibers of having a lower melting point. The specific polyester fibers used are identified as Type 676 manufactured and sold by E. I. DuPont blended with 10—15% fusible fibers. The web is heat-bonded by passing air at a temperature of about 275°F (135°C) through the web for a few seconds. The resulting web is 25 grams per sq. meter, basis weight. The web is coated by flooding it with an aqueous solution containing 38 percent solids. The solids are 90 percent sodium acrylate and 10 percent acrylic acid. Vacuum in the amount of 1 inch of mercury (3.39 kPa) is used to withdraw the excess solution from the web. The web is then subjected to 6 megarads of electron beam radiation after which about 70 grams per sq. meter of polysodiumacrylate is present. The web is again flooded, subjected to vacuum treatment, and irradiated to yield a total of about 140 grams per sq. meter of polysodiumacrylate. A third time after flooding and the vacuum treatment, the web is subjected, this time, to 12 megarads of electron beam radiation to polymerize and cross-link the monomer and form polysodium-acrylate affixed to the polyester fiber. The final amount of polysodium acrylate present is about 200 grams per sq. meter. This is equivalent to about 800% dry-add-on. The coated polyester web is then corrugated according to known procedures.

After corrugation, the web is again subjected to temperatures in the range of 275°F (135°C) so as to again make the fusible fibers soft to provide bonding between the corrugations. The corrugations are about 3/4 of an inch (1.9 cm) high and constitute approximately 4 per inch (2.54 cm) of corrugated web. A section of corrugated web 3 inches by 7 inches (7.62 × 17.78 cm) will absorb up to 150 milliliters of liquid.

Example II

An absorbing layer is formed of polyester fibers by dry laying the fibers i.e. by air-laying or carding to form a web. Specifically the polyester fibers contain a minor portion, about 10 to 15% by weight, of fusible fibers which soften at a lower temperature than the rest of the fibers. The specific polyester fibers used are identified as Type 676 fibers manufactured and solid by E. I. DuPont Company. Acrylic fibers are deposited onto the polyester web to form a discrete but gently-united layer of acrylic fibers. The two layer flexible longitudinal web is subjected to corrugating in accordance with the procedures set forth in US—A—4,111,733 and heating to a temperature of about 275°F (135°C) for a few seconds. The corrugating provides a final product having a thickness of approximately 3/4 inch (1.9 cm) weighs about 13 oz/yd$^2$ (440.8 g/m$^2$) and having approximately four corrugations per inch (2.54 cm). The corrugations are provided transversely to the web.

Example III

A disposable diaper similar to that depicted in Figure 7 is assembled using a polyethylene backing sheet and a polyester nonwoven fabric as the facing sheet. The absorbent core is provided by a section of the absorbent product of Example I measuring 8 inches wide and 13 inches long (20.32 × 33.02 cm). The diaper is assembled by known techniques utilizing hot-melt adhesive to adhere the absorbent core to the backing, and the facing and backing to each other in the side and end margins. The resulting product will contain up to at least about 200 ml of urine.

Example IV

A urinary pad to be worn by an adult is prepared by utilizing a soft, liquid-impermeable shell which is boat-like in shape. A corrugated web, like that of Example I, is prepared substantially the same except that the web is corrugated to a height of one inch (2.54 cm). A section of corrugated web is shaped by cutting it to fit into the shell (see Figure 8) so that the corrugations run lengthwise. The corrugated web section is placed in the shell with the polyester web side up and a polyester liquid-permeable facing is placed over the corrugated web and adhered to the edge of the shell. Suitable means for adhering the bottom of the shell to the user's underclothing is provided. When being used, the urinary pad is placed so that the wide portion is at the front of the crotch. The urinary pad will contain at least about 200 ml of urine.

Example V

A fibrous web is formed of polyester fibers, the fibers being staple fibers except for about 10—15% of fusible polyester fibers, the latter having a melting point of about 275°F (135°C). The web is formed by carding the fibers. The web is heat-bonded lightly by passing air, having a temperature of about 275°F (135°C), through the web for a few seconds. The resulting web is about 25 grams per sq. meter basis weight. The specific polyester fibers used are identified as type 676 Dacron, and type 581 Dacron fibers manufactured and sold by E. I. DuPont & Company. The flexible longitudinal web is subjected to corrugating in accordance with the procedure set forth in US—A—4,111,733 and subjected to heat to a temperature of about 275°F (135°C) during or subsequent to corrugation in order to again render the fusible fibers sufficiently tacky to adhere to themselves and to the staple fibers of the web in order to stabilize the corrugated web.

The corrugating provides a final product having a thickness of approximately 3/4 inch (1.9 cm) and which weighs about 13 oz. per yd. (440.8 g/m$^2$) in its corrugated form. There are approximately four corrugations per inch (2.54 cm). A powdered superabsorbent polymer is uniformly sprinkled into the pockets created by the corrugation of the nonwoven polyester fibrous web. The superabsorbent is present

at a concentration of about 400 grams per sq. meter of web. The particular superabsorbent used is identified as Permasorb #10, manufactured by National Starch and Chemical Corporation. The corrugated fiber web containing the superabsorbent is placed on a polyethylene film wherein the film extends at least one inch (2.54 cm) beyond the corrugated web on each side of the web. Polyester facing in the form of a nonwoven polyester fiber web having a weight of about 0.7 oz./sq. yd. (23.7 g/m$^2$) is placed over the corrugated web and extends beyond it on all sides by at least one inch (2.54 cm). The polyethylene backing and the polyester facing are adhered to each other by use of hot melt adhesive on all four sides to provide a unitary disposable absorbent product. The product, when prepared in a size of approximately 12 in. by 12 in. (30.5 × 30.5 cm), will hold up to at least about 200 milliliters of urine when the urine is discharged onto the facing at least about 3 inches (7.62 cm) from the edge of the product.

**Claims**

1. An absorbent product comprising:
a corrugated non-woven web comprising hydrophobic, resilient, synthetic fibers (32), stabilised to retain its transverse folds when wet, and
at least 10% by weight of the web of an absorbent (34) associated with the web,
characterised in that:
the web (32) is stabilised by face-to-face bonding (36) between corrugations; and
the absorbent is a superabsorbent (34).

2. The product of claim 1, wherein the superabsorbent (34) is present in an amount between 10 and 150% by weight of the web (32).

3. The product of claim 2, wherein the superabsorbent (34) is present in an amount between 50 and 90% by weight of the web (32).

4. The product of any one of claims 1 to 3, wherein the superabsorbent (34) is substantially affixed to the fibers of the web.

5. The product of any one of claims 1 to 3, wherein the superabsorbent (64) is placed in the transverse folds of the web (62).

6. The absorbent product of any one of claims 1 to 5, including in association with the web (126B), a second layer (128B) which has a higher capillary pressure than that of the web (126B).

7. The product of claim 6, wherein the second layer (128B) is discrete from but united to, and substantially co-extensive with, the web (126B), the second layer (128B) being denser than the web (126B) to provide preferential wicking of liquid in the second layer (128B).

8. The absorbent product of claim 6 or claim 7, wherein the superabsorbent is between the transverse folds and in contact with the second layer (128B).

9. The absorbent product of claim 6 or claim 7, wherein the superabsorbent is between the web and said second layer.

10. An absorbent product comprising a first layer (22A) and a second layer (24A) discrete from but united to said first layer (22A), wherein:
the first layer comprises a non-woven web comprising hydrophobic, resilient, synthetic fibers;
the second layer (24A) has a higher capillary pressure than that of the first layer (22A) to provide preferential attraction and wicking of liquid in the second layer (24A);
the layers are transversely folded to provide a corrugated structure; and
the corrugated structure is stabilised to retain its transverse folds when wet,
characterised in that:
the web (22A) is stabilised by face-to-face bonding between corrugations.

11. The product of any one of claims 1 to 10, wherein the web is face-to-face bonded by adhesive.

12. The product of any one of claims 1 to 10, wherein the web is face-to-face bonded by thermal bonding of heat fusible fibers contained in the web.

13. The product of any one of claims 1 to 12 wherein the web (22A) prior to corrugation has a dry bulk of at least 10 cc per gram and a weight less than 135.6 g/m$^2$ (4 ounces per square yard).

14. The product of any one of claims 1 to 13, wherein the web (22A) has a dry bulk recovery of at least 30%.

15. The product of any one of claims 1 to 14, wherein the fibers of the web (22A) are polyethylene, polyester, polypropylene or polyamide fibers or a mixture thereof.

16. The product of any of claims 1 to 14, wherein the fibers of the web (22A) are bicomponent fibers.

17. The product of claim 6 or claim 10 or any claim dependent thereon, wherein the second layer (24A) is chemically delignified wood pulp fibers, peat moss, acrylic fibers or rayon fibers or a mixture thereof.

18. The product of claim 17, wherein the web (22A) is a layer of non-woven polyester fibers and the second layer (24A) is of chemically delignified wood pulp fibers.

19. The product of any one of claims 1 to 18, further comprising a liquid impermeable barrier (104) covering at least one side of the corrugated structure and a liquid-permeable facing (102) covering at least the other side of the corrugated structure.

20. The product of claim 19 when dependent on any one of claims 1 to 3 and 6 to 8, wherein the superabsorbent is placed between the corrugated structure and the liquid-impermeable barrier (124B).

21. The product of claim 19 or claim 20 when dependent on any one of claims 1 to 3 and 6 to 8, wherein the superabsorbent is affixed to the liquid-impermeable barrier.

22. The product of any one of claims 19 to 21 in the form of a disposable diaper (80), a urinary pad (90) or a sanitary napkin (110).

**Patentansprüche**

1. Absorbierendes Produkt, umfassend:

eine gewellte Faservliesstoffbahn (32) mit einem Gehalt an hydrophoben, elastischen, synthetischen Fasern, welche Faservliesstoffbahn stabilisiert ist, um ihre Falten in der Querrichtung im nassen Zustand beizubehalten; und

wenigstens 10 Gew.-% der Bahn an einem mit der Bahn verbundenen, absorbierenden Material (34); dadurch gekennzeichnet,

daß die Bahn (32) durch direkte Bindungen (36) zwischen den Wellungen stabilisiert ist; und

daß das absorbierende Material ein superabsorbierendes Material (34) ist.

2. Produkt nach Anspruch 1, worin das superabsorbierende Material (34) in einer Menge zwischen 10 und 150 Gew.-% der Bahn (32) vorliegt.

3. Produkt nach Anspruch 2, worin das superabsorbierende Material (34) in einer Menge zwischen 50 und 90 Gew.-% der Bahn (32) vorliegt.

4. Produkt nach einem der Ansprüche 1 bis 3, worin das superabsorbierende Material (34) im wesentlichen an den Fasern der Bahn befestigt ist.

5. Produkt nach einem der Ansprüche 1 bis 3, worin das superabsorbierende Material (64) in den Querfalten der Bahn (62) angeordnet ist.

6. Absorbierendes Produkt nach einem der Ansprüche 1 bis 5, welches, in Verbindung mit der Bahn (126B), eine zweite Lage (128B) enthält, welche einen höheren Kapillardruck als jenen der Bahn (126B) aufweist.

7. Produkt nach Anspruch 6, worin die zweite Lage (128B) von der Bahn (126B) getrennt, jedoch mit derselben verbunden ist, und im wesentlichen die gleichen Abmessungen wie die Bahn (126B) aufweist, wobei die zweite Lage (128B) dichter als die Bahn (126B) ist, um für ein bevorzugtes, dochtartiges Aufsaugen von Flüssigkeit in die zweite Lage (128B) zu sorgen.

8. Absorbierendes Produkt nach Anspruch 6 oder Anspruch 7, worin das superabsorbierende Material zwischen den Querfalten und in Berührung mit der zweiten Lage (128B) vorliegt.

9. Absorbierendes Produkt nach Anspruch 6 oder Anspruch 7, worin das superabsorbierende Material zwischen der Bahn und der zweiten Lage vorliegt.

10. Absorbierendes Produkt, umfassend eine erste Lage (22A) und eine zweite Lage (24A), welche von der ersten Lage (22A) getrennt, jedoch mit derselben verbunden ist, worin:

die erste Lage eine Faservliesstoffbahn mit einem Gehalt an hydrophoben, elastischen, synthetischen Fasern umfaßt;

die zweite Lage (24A) einen höheren Kapillardruck als jenen der ersten Lage (22A) hat, um für ein bevorzugtes Anziehen und dochtartiges Aufsaugen von Flüssigkeit in die zweite Lage (24A) zu sorgen;

die Lagen unter Bildung einer gewellten Struktur in der Querrichtung gefaltet sind; und

die gewellte Struktur stabilisiert ist, um ihre Querfalten im nassen Zustand beizubehalten;

dadurch gekennzeichnet,

daß die Bahn (22A) durch direkte Bindungen zwischen den Wellungen stabilisiert ist.

11. Produkt nach einem der Ansprüche 1 bis 10, worin die Bahn direkte Klebstoffbindungen aufweist.

12. Produkt nach einem der Ansprüche 1 bis 10, worin die Bahn aufgrund des thermischen Bindens von warmverschweißbaren Fasern, die in der Bahn enthalten sind, direkt gebunden ist.

13. Produkt nach einem der Ansprüche 1 bis 12, worin die Bahn (22A) vor dem Wellen ein spezifisches Trockenvolumen von wenigstens 10 $cm^3/g$ und ein Flächengewicht von weniger als 135,6 $g/m^2$ (4 Unzen je Quadratyard) aufweist.

14. Produkt nach einem der Ansprüche 1 bis 13, worin die Bahn (22A) ein Rückstellvermögen des spezifischen Trockenvolumens von wenigstens 30% aufweist.

15. Produkt nach einem der Ansprüche 1 bis 14, worin die Fasern der Bahn (22A) Polyethylen-, Polyester-, Polypropylen- oder Polyamidfasern, oder ein Gemisch davon, sind.

16. Produkt nach einem der Ansprüche 1 bis 14, worin die Fasern der Bahn (22A) Zweikomponentenfasern sind.

17. Produkt nach Anspruch 6 oder Anspruch 10, oder nach einem darauf rückbezogenen Anspruch, worin die zweite Lage (24A) aus chemisch delignifizierten Holzzellstoffasern, Torfmoos, Acrylfasern oder Reyonfasern, oder einem Gemisch davon, besteht.

18. Produkt nach Anspruch 17, worin die Bahn (22A) eine Lage aus ungewebten Polyesterfasern ist, und die zweite Lage (24A) aus chemisch delignifizierten Holzzellstoffasern besteht.

19. Produkt nach einem der Ansprüche 1 bis 18, welches weiterhin eine flüssigkeitsundurchlässige Sperrschicht (104), welche wenigstens eine Seite der gewellten Struktur bedeckt, und eine flüssigkeitsdurchlässige Abdecklage (102), welche wenigstens die andere Seite der gewellten Struktur bedeckt, umfaßt.

20. Produkt nach Anspruch 19, sofern dieser Anspruch auf einen der Ansprüche 1 bis 3 und 6 bis 8 rückbezogen ist, worin das superabsorbierende Material zwischen der gewellten Struktur und der flüssigkeitsundurchlässigen Sperrschicht (124B) angeordnet ist.

21. Produkt nach Anspruch 19 oder Anspruch 20, sofern diese Ansprüche auf einen der Ansprüche 1 bis 3 und 6 bis 8 rückbezogen sind, worin das superabsorbierende Material an der flüssigkeitsundurchlässigen Sperrschicht befestigt ist.

22. Produkt nach einem der Ansprüche 19 bis 21, in der Form einer Wegwerfwindel (80), einer Harnaufnahmeeinlage (90) oder einer Damenbinde (110).

**Revendications**

1. Un produit absorbant comprenant:
un tissu non tissé ondulé (32) comprenant des fibres synthétiques élastiques hydrophobes, stabilisé pour maintenir ses plis transversaux lorsqu'il est mouillé, et
au moins 10% en poids du tissu d'un absorbant (34) associé au tissu,
caractérisé en ce que:
le tissu (32) est stabilisé par liaison face à face (36) entre le plis;
et l'absorbant est un superabsorbant (34).

2. Le produit selon la revendication 1 dans lequel le superabsorbant (34) est présent dans des quantités comprises entre 10 et 150% en poids du tissu (32).

3. Le produit selon la revendication 2, dans lequel le superabsorbant (34) est présent dans des quantités comprises entre 50 et 90% en poids du tissu (32).

4. Le produit selon l'une des revendications 1 à 3, dans lequel le superabsorbant (34) est substantiellement fixé aux fibres du tissu.

5. Le produit selon l'une des revendications 1 à 3, dans lequel le superabsorbant (64) est placé dans les plis transversaux du tissu (62).

6. Le produit absorbant selon l'une des revendications 1 à 5, comprenant, en association avec le tissu (126B), une seconde couche (128B) qui a une pression capillaire supérieure à celle du tissu (126B).

7. Le produit selon la revendication 6, dans lequel la seconde couche (128B) est séparée mais reliée au tissu (126B), et substantiellement de même étendue que ce dernier, la seconde couche (128B) étant plus dense que le tissu (126B) pour fournir un déplacement préférentiel du liquide au moyen de mèches dans la seconde couche (128B).

8. Le produit absorbant selon la revendication 6 ou 7, dans lequel le superabsorbant est placé entre les plis transversaux et est en contact avec la seconde couche (128B).

9. Le produit absorbant selon la revendication 6 ou 7, dans lequel le superabsorbant est placé entre le tissu et ladite seconde couche.

10. Un produit absorbant comprenant une première couche (22A) et une seconde couche (24A) séparée mais reliée à ladite première couche (22A), dans lequel:
la première couche comprend un tissu non tissé comprenant des fibres synthétiques élastiques hydrophobes;
la seconde couche (24A)a une pression capillaire supérieure à celle de la première couche (22A) pour fournir une attraction préférentielle et le déplacement du liquide au moyen de mèches dans la seconde couche (24A);
les couches sont pliées transversalement pour fournir une structure ondulée; et
la structure ondulée est stabilisée pour maintenir ses plis transversaux lorsqu'elle est mouillée,
caractérisée en ce que:
le tissu (22A) est stabilisé par liaison face à face entre les plis.

11. Le produit selon l'une des revendications 1 à 10, dans lequel le tissu est lié face à face par de l'adhésif.

12. Le produit selon l'une des revendications 1 à 10, dans lequel le tissu est lié face à face par liaison thermique de fibres fusibles contenues dans le tissu.

13. Le produit selon l'une des revendications 1 à 12, dans lequel le tissu (22A) a, avant le plissage, un volume sec d'au moins 10 $cm^3$ par gramme et un poids inférieur à 135,6/$m^2$ (4 onces par $yard^2$).

14. Le produit selon l'une des revendications 1 à 13, dans lequel le tissu (22A) a une capacité a retrouver son volume sec d'au moins 30%.

15. Le produit selon l'une des revendications 1 à 14, dans lequel les fibres du tissu (22A) sont des fibres de polyéthylène, de polyester, de polypropylène ou de polyamides ou un mélange de celles-ci.

16. Le produit selon l'une des revendications 1 à 14, dans lequel les fibres du tissu (22A) sont des fibres à deux composants.

17. Le produit selon la revendication 6 ou 10 ou toute revendication dépendante de celle-ci, dans lequel la seconde couche (24A) est composée de fibres de pulpe de bois délignifiées de façon chimique, de sphaigne, de fibres acryliques ou de fibres de rayonne ou d'un mélange de celles-ci.

18. Le produit selon la revendication 17, dans lequel le tissu (22A) est une couche de fibres de polyester non tissées et la seconde couche (24A) est composée de fibres de pulpe de bois délignifiées de façon chimique.

19. Le produit selon l'une des revendications 1 à 18, comprenant de plus une couche de barrière (104) imperméable aux liquides couvrant au moins un côté de la structure ondulée et une couche interne perméable aux liquides (102) couvrant au moins l'autre côté de la structure ondulée.

20. Le produit selon la revendication 19 quand celle-ci est dépendante de l'une des revendications 1 à 3 et 6 à 8, dans lequel le superabsorbant est placé entre la structure ondulée et la barrière imperméable aux liquides (124B).

21. Le produit selon la revendication 19 ou 20 quand celle-ci est dépendante de l'une des revendications 1 à 3 et 6 à 8, dans lequel le superabsorbant est fixé à la barrière imperméable aux liquides.

22. Le produit selon l'une des revendications 19 à 21 sous la forme d'une couche jetable pour bébé (80), d'un tampon pour incontinent (90) ou d'une serviette hygiénique (110).

FIG-1

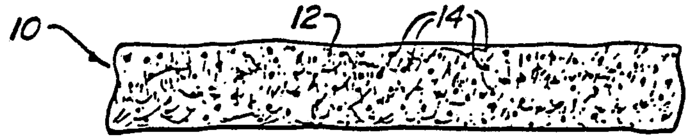


FIG-2

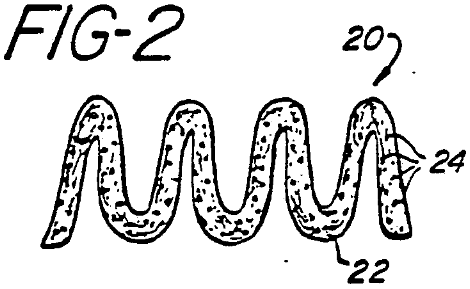


FIG-3

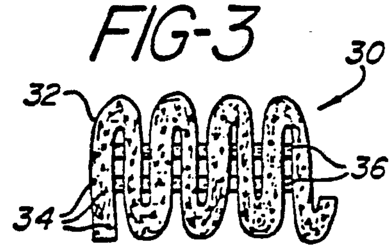


FIG-4

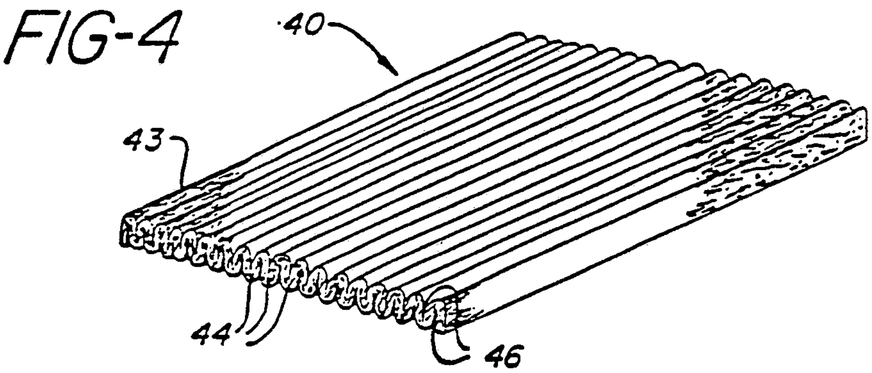


FIG-5

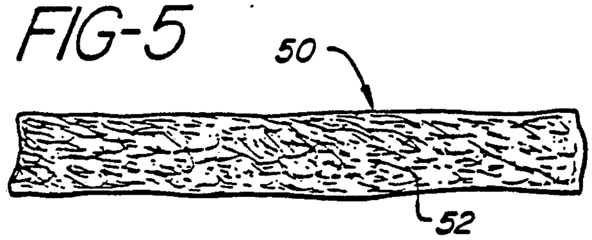

FIG-1A

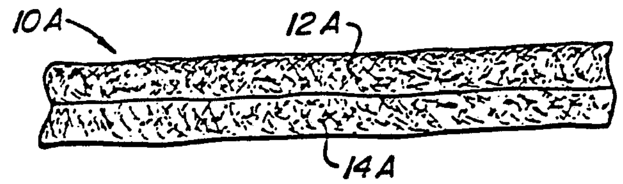


FIG-2A

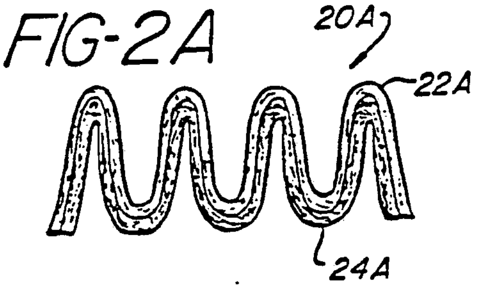


FIG-6

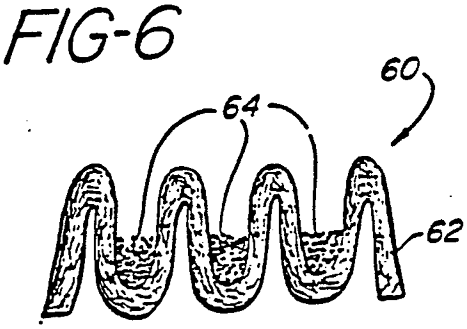


FIG-7

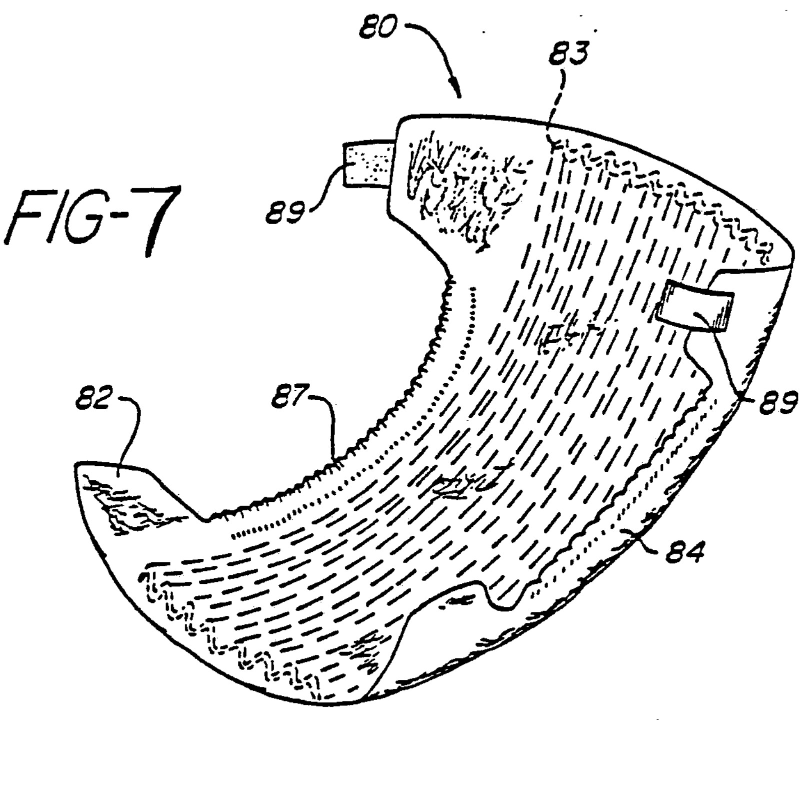

FIG-8

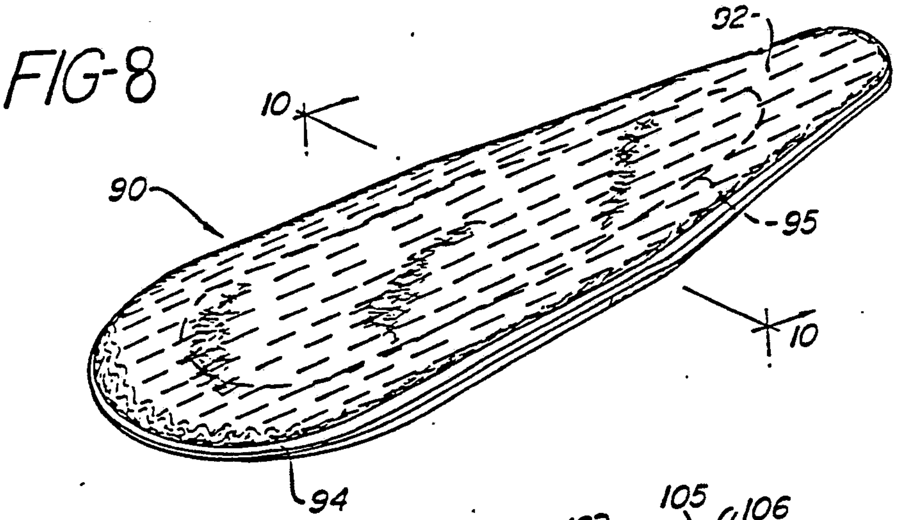


FIG-9

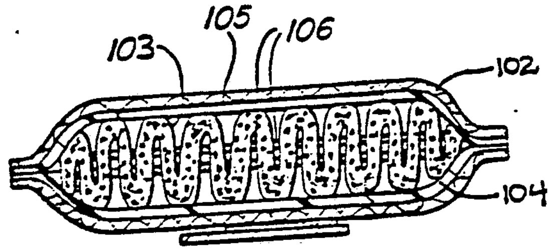


FIG-10

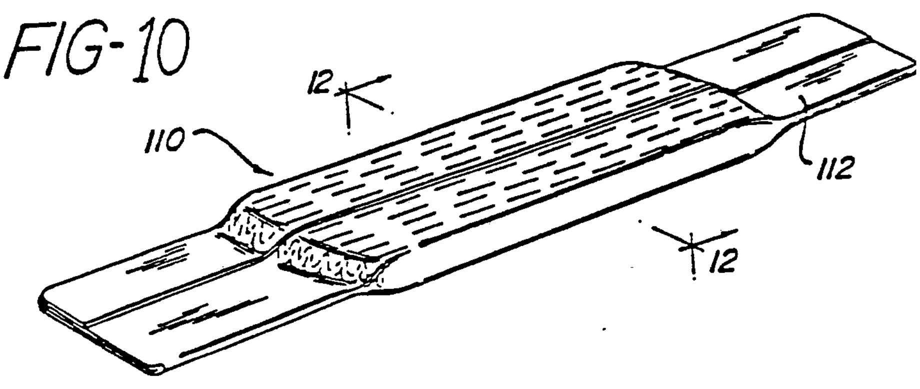


FIG-11